# EUROPEAN PATENT SPECIFICATION

(11) **EP 1 624 878 B1**
(45) Date of publication and mention of the grant of the patent: **27.09.2006**
(21) Application number: 04734005.4
(22) Date of filing: 19.05.2004
(51) Int. Cl.: A61K 31/565, A61P 19/02

(54) **USE OF COMPOSITIONS COMPRISING AN ESTROGENIC COMPONENT FOR THE TREATMENT AND PREVENTION OF MUSCULOSKELETAL PAIN**
VERWENDUNG VON ZUSAMMENSETZUNGEN ENTHALTEND EIN OESTROGEN ZUR BEHANDLUNG UND VERHINDERUNG VON MUSKELSKELETTSCHMERZEN
UTILISATION DES COMPOSITIONS COMPRENANT UN COMPOSE ESTROGEN POUR LE TRAITMENT E LA PREVENTION DE LA DOULEUR MUSCULOSQUELETTIQUE

(30) Priority: 22.05.2003 EP 03076555
(43) Date of publication of application: 15.02.2006
(73) Proprietor: Pantarhei Bioscience B.V., 3701 CH Zeist (NL)
(72) Inventor: COELINGH BENNINK, Herman, Jan Tijmen, NL-2202 DA Noordwijk (NL); BUNSCHOTEN, Evert, Johannes, NL-5384 EV Heesch (NL)
(74) Representative: van Westenbrugge, Andries
(86) International application number: PCT/NL2004/000354
(87) International publication number: WO 2004/103377

(56) References cited:
- WO-A-01/64193
- RU-C- 2 180 570

## Description

### TECHNICAL FIELD OF THE INVENTION

The present invention is concerned with the use of an estrogenic component in the manufacture of a pharmaceutical composition for treating or preventing musculoskeletal pain in a mammal. More particularly, the present invention is concerned with compounds for treating or preventing musculoskeletal pain in a mammal receiving administration of an estrogen suppressant selected from the group consisting of aromatase inhibitors, GnRH analogues, cyclo-oxygenase 2 (COX-2) inhibitors, 17β-hydroxysteroid dehydrogenase type 1 inhibitors, progestogens, anti-estrogens and combinations thereof.

### BACKGROUND OF THE INVENTION

Breast cancer is one of the most prevalent types of cancer. Epidemiological and clinical studies have shown that up till 60% of breast tumours in premenopausal women express estrogen receptors and approximately 80% of breast tumours in postmenopausal women are estrogen-sensitive. This means that estrogens are required for the growth of such breast tumours in both premenopausal and postmenopausal patients. *In situ* formation of estrogen from estrogen biosynthetic precursors within tumours is now known to make a major contribution to the estrogen content of breast tumours.

Numerous other estrogen-sensitive conditions, disorders and diseases have been identified as well, including, but not limited to ovarian and uterine cancers, melanoma, benign prostatic hyperplasia, uterine fibroids and endometriosis.

The biological effect of estrogen in the breast and other tissues is mediated by the estrogen receptor (ER), which is a member of a large family of ligand-inducible transcription factors. Upon binding to its receptor, the ligand initiates the dissociation of heat shock proteins from the receptor, receptor dimerization, phosphorylation, and binding to DNA response elements of target genes. After binding to DNA, ER differentially regulates transcription of target genes with or without other transcription factors and coactivators/corepressors.

Drugs that competitively block estrogen binding to its receptor, termed anti-estrogens, are capable of inhibiting the stimulatory effects of the hormone on cell proliferation and are therefore useful in the clinical treatment of breast cancer. Clinically, estrogen receptor-positive tumours respond with a higher frequency to anti-estrogens than do tumours lacking a significant level of receptors.

An alternative method of preventing the stimulatory effect of estrogen on cell proliferation is treatment with an aromatase inhibitor. Aromatase is one of the P-450 enzymes. It catalyses the aromatisation of the A ring of the steroid skeleton in the steroid biosynthetic pathway starting from the cleavage of the side chain of cholesterol. To be more precise: aromatase catalyses the conversion of androstenedione to estrone as well as the conversion of testosterone to estradiol. Hence aromatase is a rate limiting enzyme for the biosynthesis of the latter estrogens. Aromatase inhibitors are substances capable of inhibiting the catalytic activity of aromatase and that may be administered in non-toxic dosages so as to inhibit estrogen biosynthesis.

Treatment of estrogen sensitive tumours, such as breast cancer, with aromatase inhibitors is gaining increasing attention. The results of the so called ATAC (anastrazole, tamoxifen, alone or in combination) trial, a study in 9366 patients, demonstrated that anastrozole was significantly superior to tamoxifen for the treatment of postmenopausal women with early breast cancer with regard to disease-free survival and incidence of contralateral breast cancer (Lancet 2002 Jun 22;359(9324):2131-9). In addition, anastrozole was shown to be significantly better tolerated than tamoxifen with respect to endometrial cancer, vaginal bleeding/discharge, ischaemic cerebrovascular events, thromboembolic events and hot flushes. However, in comparison with tamoxifen, anastrozole was associated with significantly higher incidence of musculoskeletal disorders (defined as skeletal pain, pain in the legs, arms or back) and fractures.

Reduction of estrogen concentrations in blood serum may also be achieved through administering high doses of progestogen, GnRH analogue, cyclo-oxygenase 2 (COX-2) inhibitors or 17β-hydroxysteroid dehydrogenase type 1 inhibitors. However, as is the case for aromatase inhibitors, also long term suppression of endogenous estrogen production through administration of these drugs is associated with pronounced side-effects.

### SUMMARY OF THE INVENTION

The inventors have unexpectedly discovered that a very undesirable side-effect of treatment with estrogen suppressants, i.e. musculoskeletal pain, can be treated or prevented effectively by administering an estrogenic substance that is represented by the following formula: 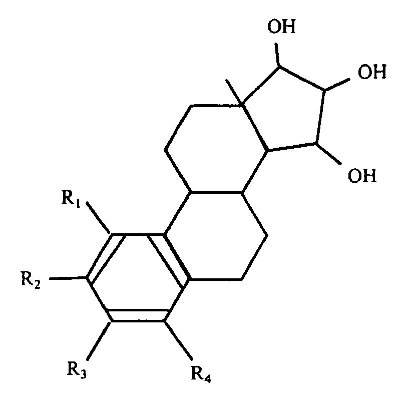 in which formula R₁, R₂, R₃, R₄ independently are a hydrogen atom, a hydroxyl group or an alkoxy group with 1-5 carbon atoms.

A known representative of this group of estrogenic substances is 1,3,5 (10)-estratrien-3,15α,16α,17β-tetrol, also known by the names of estetrol, oestetrol and 15α-hydroxyestriol. Estetrol is an estrogen that is produced by the fetal liver during human pregnancy. Unconjugated estetrol levels in maternal plasma peak at about 1.2 ng/ml at term pregnancy and are about 12 times higher in fetal than in maternal plasma (Tulchinsky et al., 1975. J. Clin. Endocrinol. Metab., 40, 560-567).

It is very surprising that the present estrogenic substances can advantageously be used in the treatment or prevention of musculoskeletal pain in patients who suffer from an estrogen-sensitive disorder without counteracting the anti-proliferative effect of the estrogen suppressant as the skilled person expects estrogenic substances to enhance cell proliferation. Since the present estrogenic substances do not appear to exhibit estrogen antagonistic properties, this finding is truly unexpected.

The present estrogenic substances were found to exhibit a relatively high affinity for the ERα receptor, or conversely a relatively low affinity for the ERβ receptor. It is believed that this receptor specificity is somehow associated with the efficacy of the present estrogenic substances in the method of the invention and in particular with the very low or non-existing proliferative activity of these substances. However, the mechanisms that govern the ER signalling pathways that are responsible for this efficacy are as yet poorly understood, despite the considerable scientific effort that is ongoing in this area. Nonetheless, it is evident that this unique specificity may well explain why the present estrogenic component, unlike commonly used estrogens, can suitable be used to treat or prevent musculoskeletal pain in patients who receive estrogen suppressant as part of the treatment of estrogen-sensitive tumours.

### DETAILED DESCRIPTION OF THE INVENTION

Accordingly, one aspect of the invention relates to a method of treating or preventing musculoskeletal pain in a mammal receiving administration of an estrogen suppressant selected from the group consisting of aromatase inhibitors, GnRH analogues, cyclo-oxygenase 2 (COX-2) inhibitors, 17β-hydroxysteroid dehydrogenase type 1 inhibitors, progestogens, anti-estrogens and combinations thereof, said method comprising the administration of an effective amount of an estrogenic component, wherein the estrogenic component is selected from the group consisting of:
substances represented by the following formula: 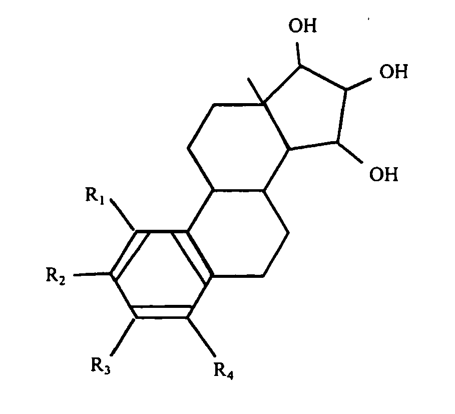
in which formula R₁, R₂, R₃, R₄ independently are a hydrogen atom, a hydroxyl group or an alkoxy group with 1-5 carbon atoms; precursors capable of liberating a substance according to the aforementioned formula when used in the present method; and mixtures of one or more of the aforementioned substances and/or precursors. Naturally, the advantages offered by the present invention may also be realised by employing a metabolite of the above mentioned estrogenic substances, even though it is preferred to employ said estrogenic substances.

The term "musculoskeletal pain" refers to a pronounced pain sensation in the muscles of legs, arms or back or in the skeleton, especially the joints. The term musculoskeletal pain does not encompass osteoporosis which is a well-known side effect of prolonged treatment with estrogen suppressants.

As used herein the term "tumour" refers to a new growth of tissue in which the multiplication of cells is uncontrolled and progressive. The term tumour encompasses both malignant tumours (e.g. breast cancer) and benign tumours (e.g. endometriosis).

The term "estrogen-sensitive tumour" refers to a tumour whose formation and growth is stimulated by estrogens, other than the estrogenic components according to the present invention, especially 17β-estradiol, ethinyl estradiol, as well as precursors and metabolites thereof.

The term "cancer" as used throughout this document refers to cells that have undergone a malignant transformation that makes them pathological to the host organism.

The present estrogenic substances are distinct from both the biogenic and synthetic estrogens that are commonly applied in pharmaceutical formulations in that the 5 membered ring in the steroid skeleton comprises 3 hydroxyl substituents rather than 0-2. In a particularly preferred embodiment at least one of R₁, R₂, R₃ and R₄ represents a hydroxyl group, meaning that the estrogen substance contains at least 4 hydroxyl groups. Preferably, the estrogenic component applied as the active component in the present composition is a so called biogenic estrogen, i.e. an estrogen that occurs naturally in the human body, a precursor of a biogenic estrogen or a mixture thereof. Because biogenic estrogens are naturally present in the fetal and female body, side-effects are not expected to occur, particularly not if the serum levels resulting from the exogenous administration of such estrogens do not substantially exceed naturally occurring concentrations.

In a preferred embodiment of the present invention the estrogenic substance contains 4 hydroxyl groups. In another preferred embodiment, no more than 3 of R₁, R₂, R₃, R₄ are hydrogen atoms. Also, in the aforementioned formula, R₁ preferably represents a hydrogen atom. In said formula preferably at least 2, more preferably at least 3 of the groups R₁, R₂, R₃ and R₄ represent a hydrogen atom.

The estrogenic substances according to the formula encompass various enantiomers since the carbon atoms that carry hydroxyl-substituents are chirally active. In one preferred embodiment, the present estrogenic substance is 15α-hydroxy substituted. In another preferred embodiment the substance is 16α-hydroxy substituted. In yet another preferred embodiment, the substance is 17β-hydroxy substituted. Most preferably the estrogenic substances are 15α,16α,17β-trihydroxy substituted. The other chirally active carbon atoms in the steroid skeleton of the present estrogenic components preferably have the same configuration as the corresponding carbon atoms in 17β-estradiol and other biogenic estrogens.

In a preferred embodiment of the present invention R₃ represents a hydroxyl group or an alkoxy group. In another preferred embodiment the groups R₁, R₂ and R₄ represent hydrogen atoms, in which case the substance is 1,3,5 (10)-estatrien-3, 15,16,17-tetrol. A preferred isomer of the latter substance is 1,3,5 (10)-estatrien-3, 15α,16α,17β-tetrol (estetrol).

The invention also encompasses the use of precursors of the estrogen substances that constitute an active component in the present method. These precursors are capable of liberating the aforementioned estrogen substances when used in the present method, e.g. as a result of metabolic conversion. These precursors are preferably selected from the group of derivatives of the present estrogen substances, wherein the hydrogen atom of at least one of the hydroxyl groups has been substituted by an acyl radical of a hydrocarbon carboxylic, sulfonic acid or sulfamic acid of 1-25 carbon atoms; tetrahydrofuranyl; tetrahydropyranyl; or a straight or branched chain glycosydic residue containing 1-20 glycosidic units per residue. Typical examples of precursors which can suitably be used in accordance with the invention are esters that can be obtained by reacting the hydroxyl groups of the estrogen substances with substances that contain one or more carboxy (M⁺ -OOC-) groups, wherein M⁺ represents a hydrogen or (akali)metal cation. Hence, in a particularly preferred embodiment, the precursors are derivatives of the estrogen substances, wherein the hydrogen atom of at least one of the hydroxyl groups in said formula has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

The present method may suitably be used to treat or prevent musculoskeletal pain that is associated with pharmaceutical treatments that are based on an estrogen suppressant, in particular with treatments that employ uninterrupted administration of an estrogen suppressant during a period of at least 1 month, particularly at least 2 months and most particularly at least 4 months. Estrogen suppressants are commonly employed to prevent interaction between endogenous estrogens, notably 17β-estradiol and estrogen receptors in tissues that are sensitive to estrogen-induced proliferation. This may suitably be achieved by "blocking" the estrogen receptor with an anti-estrogen such as tamoxifen, or by preventing endogenous production of 17β-estradiol and estrone with the help of an aromatase inhibitor, progestogen, GnRH analogue, cyclo-oxygenase 2 (COX-2) inhibitors or 17β-hydroxysteroid dehydrogenase type 1 inhibitors.

The biosynthetic pathways that are involved in the endogenous production of the most important biogenic estrogen, i.e. 17β-estradiol, may be represented as follows:

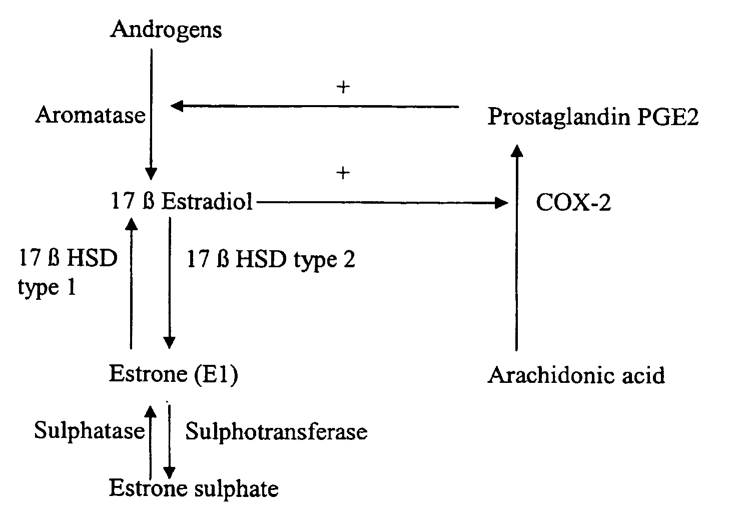

As is evident from the above diagram, aromatase and 17β-hydroxysteroid dehydrogenase type 1 are key enzymes in the endogenous production of 17β-estradiol. Consequently, the inhibition of aromatase and 17β-hydroxysteroid dehydrogenase type 1 in tissues will automatically reduce the production of 17β-estradiol therein.

The diagram also shows that prostaglandin PGE2 is capable of stimulating aromatase activity. Consequently, inhibition of cyclo-oxygenase 2 (COX-2), the enzyme responsible for the endogenous production of PGE2 from arachidonic acid, will automatically cause a reduction of aromatase activity and a corresponding decrease in estrogen synthesis.

Thus, it may be concluded that aromatase inhibitors, cyclo-oxygenase 2 (COX-2) inhibitors as well as 17β-hydroxysteroid dehydrogenase type 1 inhibitors may suitably be used to impair endogenous production of estrogens.

Aromatase catalyses the aromatisation of the A ring of the steroid skeleton in the steroid biosynthetic pathway. To be more precise: aromatase catalyses the conversion of androstenedione to estrone as well as the conversion of testosterone to estradiol. Hence aromatase is a rate limiting enzyme for the biosynthesis of the latter estrogens. Aromatase inhibitors are substances capable of inhibiting the catalytic activity of aromatase. In the context of the present invention aromatase inhibitors are substances that may be administered to animals, and especially humans, in non-toxic dosages so as to inhibit estrogen biosynthesis.

At present a range of aromatase inhibitors is available and includes substances such as aminoglutethimide, anastrozole, exemestane, vorozole, letrozole, fadrozole, finrozole, rogletimide, atamestane, formestane, liarozole, YM 511, TZA-2237, CGS 16949A and MEN 11066. Aromatase inhibitors primarily find application in methods of treating breast cancer. It has also been suggested that aromatase inhibitors may be used in the treatment of endometriosis. Takayama et al. (Fertility Sterility 1998; 69(4);709-13) successfully treated one case of an unusually aggressive recurrent postmenopausal endometriosis with an aromatase inhibitor. The present method is particularly effective if the aromatase inhibitor is selected from the group consisting of anastrazole, exemestane, formestane, letrozole, fadrozole, precursors of these inhibitors and combinations thereof.

Cyclooxygenase (COX), also known as prostaglandin G/H synthase, is a membrane-bound enzyme responsible for the oxidation of arachidonic acid to prostaglandins that was first identified over 20 years ago. In the past decade, however, more progress has been made in understanding the role of cyclooxygenase enzymes in various pathophysiological conditions. Two cyclooxygenase isoforms have been identified and are referred to as COX-1 and COX-2. COX-1 enzyme is constitutively expressed and regulates a number of housekeeping functions such as vascular hemostasis and gastroprotection, whereas COX-2 is inducible (i.e., sites of inflammation) by a number of mediators such as growth factors, cytokines and endotoxins.

Nonsteroidal anti-inflammatory drugs (NSAIDs) produce their therapeutic effects through inhibition of COX, the enzyme that makes prostaglandins. Most traditional NSAIDs inhibit both COX-1 and COX-2 isoforms of cyclo-oxygenase. Non-selective inhibition of COX iso-enzyme leads to not only beneficial therapeutic effects but also a number of detrimental effects. Examples of COX-2 selective inhibitors include: celecoxib; deracoxib; valdecoxib; rofecoxib; parecoxib; etoricoxib; meloxicam; etoldolac; lumiracoxib; nimesulide; leflunomide; tilmacoxib and flosulide.

Examples of 17β-hydroxysteroid dehydrogenase type 1 inhibitors (17 β-HSD type 1 inhibitors) include: N-butyl, N-methyl, 9-[3' 17'beta-(dihydroxy)-1',3',5'(10')-estratien-16 álpha-yl]-7 bromononamide; N-butyl, N-methyl, 7-[3',17'beta-dihydroxy-1',3',5'(10')-estratiene-6' beta-yl]-7-thiaheptanamide.

Progestogens that can be used to suppress endogenous secretion of estrogens include: progesterone, levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagestone, chlormadinone, cyproterone, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone, megestrol, melengestrol, nomegestrol, norethindrone (=norethisterone), norethynodrel, norgestrel (includes d-norgestrel and dl-norgestrel), norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime. Particularly suitable are progestogens selected from the group consisting of progesterone, desogestrel, etonogestrel, gestodene, dienogest, levonorgestrel, norgestimate, norethisterone, drospirenone, trimegestone, dydrogesterone, precursors of these progestogens and mixtures thereof.

The GnRH analogues that may be used to suppress endogenous estrogen synthesis include gonadotropic horomone-releasing hormone (GnRH) as well as GnRH agonists and GnRH antagonists. Examples of suitable GnRH analogues include GnRH, buserelin. leuprorelin, goserelin, ganirelix, cetrorelix, precursors of these analogues and combinations thereof.

Anti-estrogens are substances which exhibit affinity for the mammalian estrogen receptors without triggering all of the responses that are characteristic of the interaction between estrogens and the same receptors. The term anti-estrogen as used throughout this document encompasses both anti-estrogens that trigger no estrogen receptor response at all ("true" antagonists) as well as anti-estrogens that are capable of triggering a selective estrogen receptor response.

Examples of 'true' anti-estrogens include ICI 164384, ICI 182780, ZM 189154, EM-800, RU 58668. Anti-estrogens that exert selective estrogen receptor activity include tamoxifen, raloxifene, toremifene, idoxifene, droloxifene, nafoxidine, trioxifene, MER 25, EM-652, clomiphene, cyclophenil, lasofoxifene, arzoxifene, levormeloxifene, zindoxifene, LY 117018, LY 326315, ZK 119010, LY 357489, GW 5638, GW 7604, TSE-424, FC1271a and mixtures thereof. The present method is particularly effective if the anti-estrogen is selected from the group consisting of ICI 164384, ICI 182789, raloxifene, tamoxifen, precursors of these substances, and mixtures thereof. Most preferably the anti-estrogen is selected from the group consisting of tamoxifen, ICI 164384, ICI 182789, precursors of these substances and mixtures thereof.

In a particularly preferred embodiment of the present invention, the estrogen suppressant employed is selected from the group consisting of aromatase inhibitors, GnRH analogues, cyclo-oxygenase 2 (COX-2) inhibitors, 17β-hydroxysteroid dehydrogenase type 1 inhibitors, progestogens and combinations thereof. These estrogen suppressants have in common that they are capable of inhibiting the endogenous secretion of estrogens. The benefits of the present invention are particularly pronounced when the present estrogenic component is administered to treat or prevent musculoskeletal pain that is caused by the administration of one of these inhibitors of estrogen secretion. Possibly, this is due to the fact that these inhibitors, unlike anti-estrogens, do not prevent interaction of the present estrogenic component with the estrogen receptor. In an even more preferred embodiment, the estrogen suppressant is selected from the group consisting of aromatase inhibitors, GnRH analogues, progestogens and combinations thereof.

Most preferably, the estrogen suppressant is an aromatase inhibitor. Aromatase inhibitors generally produce more severe musculoskeletal pain than anti-estrogens, progestogens cyclo-oxygenase 2 (COX-2) inhibitors, 17β-hydroxysteroid dehydrogenase type 1 inhibitors and GnRH analogues. Thus, the (co-)administration of the present estrogenic component is deemed to be particularly effective in suppressing musculoskeletal pain induced by aromatase inhibitors.

In a particularly preferred embodiment of the invention, the method comprises coadministration of an estrogen suppressant and the present estrogenic component. Such coadministration may be achieved by administering a single formulation that contains both the estrogenic component and the estrogen suppressant or, alternatively, these active principles may be administered separately. In case these active principles are administered separately, it is preferred to deliver them with the same frequency, particularly once daily. Also it is preferred to administer them essentially simultaneously. Most preferably both active principles are administered using the same mode of administration, preferably oral administration.

As mentioned herein before, aromatase inhibitors, GnRH analogues, cyclo-oxygenase 2 (COX-2) inhibitors, 17β-hydroxysteroid dehydrogenase type 1 inhibitors, progestogens may be employed to prevent the endogenous production of 17β-estradiol and estrone. In the present method these estrogen suppressants are preferably administered in an amount sufficient to reduce the serum concentration of 17β-estradiol in the mammal to less than 10 pg/ml, more preferably to less than 5 pg/ml.

According to a particularly preferred embodiment the present invention employs intravaginal or intrauterine administration of progestogen to prevent endometrial stimulation by the estrogenic component. Preferably such local administration of progestogen is combined with systemic administration of the estrogenic component. In another preferred embodiment, the method combines intravaginal or intrauterine administration of progestogen with administration of an estrogen suppressant through another route of delivery.

The present method is particularly effective when the administration is continued for a prolonged period of time. Usually, the method comprises the uninterrupted administration of the estrogenic component during a period of at least 5 days. Preferably the uninterrupted administration is continued for at least 30 days, more preferably for at least 90 days.

In accordance with the present invention, the estrogenic component may be administered enterally or parenterally. The term "parenteral administration" as used in here encompasses transdermal, intravenous, intranasal, intravaginal, pulmonary, buccal, subcutaneous, intramuscular and intra-uterine administration. The term "enteral administration" includes oral as well as rectal administration. Preferably the estrogenic component is administered orally, intranasally, transdermally, subcutaneously, intramuscularly, intravenously, intravaginally, intrauterinely, pulmonary, bucally or rectally. In a particularly preferred embodiment the estrogen suppressant and estrogenic component are administered in the same way, most preferably orally. The term oral administration as used in here also encompasses oral gavage administration.

Oral, intravenous, subcutaneous, intramuscular, intranasal, rectal, buccal and pulmonary administration are ideally suited for (at least) once daily administration. Transdermal administration is advantageously applied at frequencies between once a day and once a month. Intravaginal and intra-uterine administrations are advantageously operated at administration frequencies between once weekly and once monthly. Subcutaneous and intramuscular administration may also suitably be done in the form of depot injections at intervals of 1 week to 6 months, preferably at intervals of 4 weeks to 3 months.

For reasons of convenience, the present method preferably utilises administration intervals of 1 day, 1 week or 1 month. Regimens that employ once daily oral, subcutaneous, intravenous or intranasal administration, once weekly transdermal or once monthly intravaginal or subcutaneous administration are particularly preferred.

The estrogen suppressant employed in accordance with the present invention is preferably administered orally, intranasally, transdermally, subcutaneously, intramuscularly, intravenously, intravaginally, intrauterinely, pulmonary, rectally or buccally. In case of breast cancer it may be advantageous to deliver the estrogen suppressant locally, e.g. by means of transdermal administration.

The present method may suitably be used to treat or prevent musculoskeletal pain that is associated with (prophylactic) treatment of various estrogen-sensitive tumours, including breast cancer, uterine cancer, ovarian cancer, endometriosis, uterine fibroids, benign prostatic hyperplasia and melanoma. The term "uterine cancer" encompasses endometrial cancer and cervix cancer. The present is method is deemed to be particularly suitable for treating or preventing musculoskeletal pain in mammals suffering from breast cancer or endometrial cancer. The method of the present invention is most advantageously employed in mammals, particularly female mammals, suffering from breast cancer.

The method according to the present invention may suitably be used to treat mammals such as cattle, pets and particularly humans. The method may be used to treat both females and males (e.g. prostatic hyperplasia), be it that best results are obtained in females. The method may be applied advantageously in premenopausal, perimenopausal and postmenopausal females. Most preferably, the present method is used to treat postmenopausal females.

In another preferred embodiment, the present method is used to treat an estrogen-receptor positive patient. The term "estrogen-receptor positive patient" refers to a patient who suffers from tumours whose growth is stimulated by 17β-estradiol.

Irrespective of the mode of administration, the estrogenic component is preferably administered in an amount effective to achieve a blood serum concentration of at least 1 nanogram per litre, more preferably of at least 10 nanogram per litre, most preferably at least 100 nanogram per litre. Generally the resulting blood serum concentration of the estrogenic component will not exceed 100 µg per litre, preferably it will not exceed 50 µg per litre, more preferably it will not exceed 25 µg per litre.

In accordance with the present method the estrogenic component is usually administered in an amount of less than 1 mg per kg of bodyweight per day, preferably of less than 0.4 mg per kg of bodyweight per day. In order to achieve a significant impact from the administration of the estrogenic component, it is advisable to administer in an amount of at least 1 µg per kg of bodyweight per day. Preferably, the administered amount is at least 5 µg per kg of bodyweight per day.

Oral administration of the estrogenic component is preferably done in an amount of less than 400 µg per kg of bodyweight per day, preferably of less than 200 µg per kg of bodyweight per day. In order to achieve a significant impact from the administration of the active component, it is advisable to orally administer in an amount of at least 2 µg per kg of bodyweight per day. Preferably, the orally administered amount is at least 5 µg per kg of bodyweight per day. In the present method, particularly when used in humans, the estrogenic component is usually administered in an average dosage of at least 0.01 mg per day, preferably of at least 0.05 mg per day and most preferably of at least 0.1 mg per day. The maximum dosage is normally kept below 40 mg per day, preferably below 20 mg per day.

The present method of treatment comprises administering to a mammal in need of such a therapy an effective amount of the estrogenic component. The amount needed to be effective will differ from individual to individual and are determined by factors such as the individual's gender, body weight, route of administration and the efficacy of the particular estrogenic component used.

In the present method, particularly when used in humans, the estrogenic component is usually administered orally in an average dosage of between 0.01 and 20 mg per day, preferably of between 0.05 and 10 mg per day. Similarly, the parenteral dosage preferably is at least 0.05, preferably at least 0.1 mg per day. The average maximum parenteral dosage is normally kept below 40 mg per day, preferably below 20 mg per day.

Typically, the estrogen suppressant is administered in an amount of at least 0.01 mg. Preferably, the estrogen suppressant is administered in an average daily amount within the range of 0.2 to 3000 µg per kg of bodyweight. Preferably, it is administered in an average daily amount of 0.5-2000 µg per kg of bodyweight. In humans, the estrogen suppressant is suitably administered in an average daily amount of between 0.01 and 300 mg, preferably between 0.02 and 200 mg.

In a preferred embodiment aromatase inhibitor is administered in an amount equivalent to an oral dosage of at least 0.05 mg anastrozole, more preferably of at least 0.1 mg anastrozole and most preferably of at least 0.3 mg anastrozole. Typically, aromatase inhibitor is administered in an amount that does not exceed the equivalent of an oral dosage of 30 mg anastrozole, preferably it does not exceed the equivalent of an oral dosage of 20 mg anastrozole, more preferably of an oral dosage of 10 mg.

In another preferred embodiment, anti-estrogen is administered in an amount equivalent to an oral dosage of at least 1 mg tamoxifen, more preferably of at least 4 mg tamoxifen and most preferably of at least 6 mg tamoxifen. The anti-estrogen is generally administered in an amount that does not exceed the equivalent of an oral dosage of 200 mg tamoxifen, preferably it does not exceed the equivalent of an oral dosage of 120 mg tamoxifen, more preferably of an oral dosage of 80 mg.

The invention is further illustrated by means of the following examples.

### EXAMPLES

### Example 1

In order to assess the effect of the present estrogenic substances on tumours, estetrol was tested in the 7, 12-dimethyl-benz(a)anthracene (DMBA)-induced tumour model in rats. This model, originally developed by Huggins et al.,1961 (Nature, 19, 204-207), has been widely used and is a generally accepted model with predictive value for anti-tumour agents in humans. The growth of the DMBA-induced tumours is dependent on endogenously produced estradiol or exogenously administered estrogens and prolactin (Sylvester et al., 1982, Cancer Research, 42, 4943-4947). Ovariectomy (Hollingsworth et al., 1998, Breast Cancer Research and Treatment, 47, 63-70), androgens (Dauvois et al., 1989, Breast Cancer Treatment, 14, 299-306), tamoxifen (Hollingsworth et al., 1998, Breast Cancer Research and Treatment, 47, 63-70), progestogens (Kelly et al. 1979, Eur. J. Cancer, 15, 1243-1251; Russo et al., 1987, Lab. Invest. 57, 112-137) and GnRH analogues (Hollingsworth et al., 1998, Breast Cancer Research and Treatment, 47, 63-70) all have been shown to be effective anti-tumour treatments in the DMBA model.

Eighty-four female Sprague-Dawley rats (Harlan, The Netherlands) were group housed, maintained in a 12-hr light/dark environment, and fed a Soya Free Diet (SDS England) and water ad libitum. Animals were weighed on a weekly basis. One week prior to induction of mammary carcinoma, 12 animals (aged 43 days) were surgically castrated via removal of the ovaries. At the age of 50 days, all animals were administered a single oral dose of 16 mg DMBA to induce tumour development. Animals were subsequently allocated to one of seven groups (n=12), receiving placebo or treatment as follows:.
■ *Group 1* animals received placebo oral treatment with 3.0 ml/kg/day vehicle (20% wt/vol solution of hydroxypropyl-beta-cyclodextrin in water);
■ *Group 2* surgically castrated animals received placebo treatment with 3.0 ml/kg/day vehicle;
■ *Group 3* animals received the anti-estrogen tamoxifen given orally at a single daily dose of 3 mg/kg;
■ *Group 4* animals received ethinylestradiol (EE) orally at a single daily dose of 0.025 mg/kg;
■ *Group 5* animals received ethinylestradiol (EE) orally at a single daily dose of 0.125 mg/kg;
■ *Group 6* animals received estetrol (E4) orally at a single daily dose of 0.5 mg/kg; and
■ *Group 7* animals received estetrol (E4) orally at a single daily dose of 2.5 mg/kg.

The doses of EE and E4 were based on data from previous studies, showing equipotency of 0.025 mg/kg/day EE and 0.5 mg/kg/day E4 in agonistic models of preventing bone resorption, prevention of hot flushing and vaginal cornification. Similarly, the doses of 0.125 mg/kg/day EE and 2.5 mg/kg/day E4 showed equipotency in *in vivo* estrogenicity in preventing bone resorption, prevention of hot flushing and vaginal cornification.

During the treatment period of 8 weeks, the emergence of palpable tumours and number of tumours were determined weekly. At 8 weeks, at necropsy, final measurements were taken. The number of tumours at necropsy are depicted in figure 1. As is clearly demonstrated by the absence of tumours in the ovariectomized animals (group 2), development of DMBA-induced mammary tumours is estrogen-dependent. As expected, also tamoxifen showed anti-tumour properties by inhibiting the development of mammary tumours in this model. Surprisingly, and in contrast to the effect seen with the 0.125 mg/kg/day dose of EE, E4 at an equipotent agonistic dose of 2.5 mg/kg/day markedly suppressed mammary tumour development. Furthermore, this particular dose of E4 was as effective as tamoxifen in preventing growth of DMBA-induced tumours. 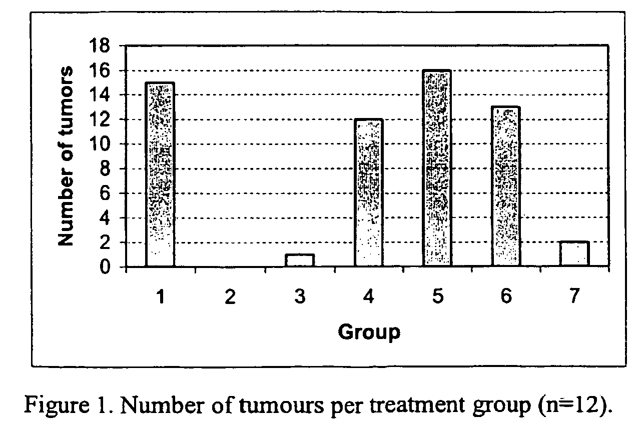
*Group 1* oral treatment with 3.0 ml/kg/day vehicle;
*Group 2* surgically castrated animals receiving placebo treatment with 3.0 ml/kg/day vehicle;
*Group 3* tamoxifen 3 mg/kg/day orally;
*Group 4* ethinylestradiol (EE) 0.025 mg/kg/day orally;
*Group 5* EE 0.125 mg/kg/day orally;
*Group 6* estetrol (E4) 0.5 mg/kg/day orally;
*Group 7* E4 2.5 mg/kg/day orally.

### Example 2

A randomised, double-blinded, placebo-controlled cross-over study is performed to show that estetrol diminishes musculoskeletal pain in postmenopausal breast cancer patients that have been receiving estrogen suppressant therapy.

20 postmenopausal patients are recruited that have breast cancer in clinical staging I or II, are adequately being treated surgically, chemotherapeutically and/or radiotherapeutically, show estrogen receptor positive tumour histology and do no have contraindications for steroid therapy, are on treatment with either an anti-estrogen or an aromatase inhibitor for at least three months and suffer from musculoskeletal pain complaints (defined as skeletal pain, pain in the legs, arms or back). Furthermore, during the screening phase other diagnoses, such as rheumatoid arthritis and/or other skeletal diseases or joint diseases, are excluded.

The patients participate in a clinical study with a duration of 15 weeks and are randomized as follows:

### Group 1 (n=10)

Patients continue with the anti-estrogen treatment that they have been receiving previously during the entire period of the study and receive additional daily oral treatment with 4 mg estetrol or a placebo for six weeks. 5 patients start with 4 mg/day oral estetrol treatment and 5 patients with placebo treatment. After a wash-out period of 3 weeks, during which anti-estrogen treatment continues and no additional estetrol or placebo treatment is given, study treatment is reinitiated. Placebo-receiving patients are subsequently treated with 4 mg/day oral estetrol and estetrol-receiving patients with placebo for 6 weeks after which the study is terminated.

### Group 2 (n=10)

Patients continue with the aromatase inhibitor treatment that they have been receiving previously during the entire period of the study and receive additional daily oral treatment with 4 mg estetrol or a placebo for six weeks. 5 patients start with 4 mg/day oral estetrol treatment and 5 patients with placebo treatment. After a wash-out period of 3 weeks, during which aromatase inhibitor treatment continues and no additional estetrol or placebo treatment is given, study treatment is reinitiated. Placebo-receiving patients are subsequently treated with 4 mg/day oral estetrol and estetrol-receiving patients with placebo for 6 weeks after which the study is terminated.

The subjects visit the study center once weekly, during which the patients are closely monitored by their study physician. During all visits, thorough physical examinations are performed, blood samples are retrieved and a questionnaire, specifically addressing musculoskeletal pain sensation on a graded scale and climacteric complaints, is completed. Primary endpoints in the study are improvement of the musculoskeletal pain and reduction of climacteric complaints, whereas secondary endpoints are clinical tumour progression and changes of tumour markers.

The results of this study show a beneficial association between estetrol administration and the severity of the musculoskeletal pain sensation as well as with the occurrence of climacteric complaints such as hot flushes. In the subset of breast cancer patients receiving concomitantly anti-estrogen therapy and estetrol the average musculoskeletal pain sensation value during treatment weeks is significantly lower than in the subset receiving concomitantly anti-estrogen therapy and placebo treatment. Similarly, in the subset of breast cancer patients receiving concomitantly aromatase inhibitor therapy and estetrol the average musculoskeletal pain sensation value during treatment weeks is significantly lower than in the subset receiving concomitantly aromatase inhibitor therapy and placebo treatment.

Furthermore, responses to the questionnaires show an overall reduction in climacteric complaints in patients receiving additional estetrol treatment. These beneficial changes tend to be most pronounced for the group of patients receiving estetrol treatment in combination with aromatase inhibitor therapy.

As compared to baseline values and tumour recurrency data for breast cancer grading stages I and II, clinical tumour progression and tumour markers do not show significant adverse changes during the treatment with estetrol. From these results it can be concluded that the combination of an estrogen suppressant and estetrol can be employed advantageously in postmenopausal breast cancer patients to prevent the occurrence of musculoskeletal pain and climacteric complaints.

### Example 3

Using the procedure as set forth in example 4, a randomised, double-blinded, placebo-controlled cross-over study is performed to show that estetrol diminishes musculoskeletal pain in premenopausal breast cancer patients receiving estrogen suppressant therapy.

In these premenopausal breast cancer patients ovarian ablation is established by either chemical, surgical, radiotherapeutical means or is reversibly induced by means of a GnRH analog. The results obtained are similar to the clinical outcome reported for postmenopausal breast cancer patients in Exmple 4. From these results it can be concluded that the combination of an estrogen suppressant and estetrol can be employed advantageously in premenopausal breast cancer patients to prevent the occurrence of musculoskeletal pain.

### Example 4

A randomised, double-blinded, placebo-controlled cross-over study is performed to show that additional estetrol treatment of postmenopausal breast cancer patients, receiving estrogen suppressant therapy, induces favourable changes in the gene expression profiles from tumours that are obtained by fine needle aspiration and diminishes musculoskeletal pain.

20 postmenopausal patients are recruited that have breast cancer in at least clinical staging III, have a palpable macroscopic tumour mass, show estrogen receptor positive tumour histology, have already been treated with either an anti-estrogen or an aromatase inhibitor for at least three months, do no have contraindications for steroid therapy and suffer from musculoskeletal pain complaints (defined as skeletal pain, pain in the legs, arms or back). Furthermore, during the screening phase other diagnoses, such as rheumatoid arthritis and/or other skeletal diseases or joint diseases, are excluded.

The patients participate in a clinical study with a duration of 15 weeks and are randomized as follows:

### Group 1 (n=10)

Patients continue with the anti-estrogen treatment they have been receiving previously during the entire period of the study and receive additional daily oral treatment with 4 mg estetrol or a placebo for six weeks. 5 patients start with 4 mg/day oral estetrol treatment and 5 patients with placebo treatment. After a wash-out period of 3 weeks, during which anti-estrogen treatment continues and no additional estetrol or placebo treatment is given, study treatment is reinitiated. Placebo-receiving patients are subsequently treated with 4 mg/day oral estetrol and estetrol-receiving patients with placebo for 6 weeks after which the study is terminated.

### Group 2 (n= 10)

Patients continue with the aromatase inhibitor treatment they have been receiving previously during the entire period of the study and receive additional daily oral treatment with 4 mg estetrol or a placebo for six weeks. 5 patients start with 4 mg/day oral estetrol treatment and 5 patients with placebo treatment. After a wash-out period of 3 weeks, during which aromatase inhibitor treatment continues and no additional estetrol or placebo treatment is given, study treatment is reinitiated. Placebo-receiving patients are subsequently treated with 4 mg/day oral estetrol and estetrol-receiving patients with placebo for 6 weeks after which the study is terminated.

The patients visit the study center once weekly, during which the patients are closely monitored by their study physician. During all visits, thorough physical examinations are performed, blood samples are retrieved and a questionnaire, specifically addressing musculoskeletal pain sensation on a graded scale, is completed. Furthermore, three fine needle aspirations (FNA) from the macroscopic tumour are obtained at baseline, during the wash-out and at the last visit after study week 15, respectively. Additionally at the same time points and for all locations with macroscopic palpable tumour activity, tumour sizes are scored. Tumour gene expression profiles are determined by using a FNA-cDNA microarray as described by Sotiriou et al. (Breast Cancer Research 2002,4:R3). Primary endpoints in the study are changes of the gene expression profile, improvement of the musculoskeletal pain and reduction of climacteric complaints, whereas secondary endpoints are clinical tumour progression and changes of tumour markers.

The results of this study show a favourable association between estetrol administration and changes in the gene-expression profile involved in the regulation and deregulation of breast tissue proliferation. Surprisingly, the concomitant administration of estetrol and either an anti-estrogen or an aromatase inhibitor synergistically produces a more beneficial molecular profile, i.e. the changes are significantly more pronounced than can be anticipated on the basis of the effects of each of the individual components alone.

Furthermore, the results of this study show a beneficial association between estetrol administration and the severity of the musculoskeletal pain sensation. In the subset of breast cancer patients receiving concomitant anti-estrogen therapy and estetrol the average musculoskeletal pain sensation value during treatment weeks is significantly lower than in the subset receiving concomitant anti-estrogen therapy and placebo treatment. Similarly, in the subset of breast cancer patients receiving concomitant aromatase inhibitor therapy and estetrol the average musculoskeletal pain sensation value during treatment weeks is significantly lower than in the subset receiving concomitant aromatase inhibitor therapy and placebo treatment.

Responses to the questionnaires show an overall reduction in climacteric complaints in patients receiving additional estetrol treatment. Moreover, these beneficial changes tend to be more pronounced for the group of patients receiving additional estetrol treatment and aromatase inhibitor therapy.

As compared to baseline values and tumour progression data for breast cancer grading stages III and more, tumours progress more slowly or sizes of macroscopically tumours decline in patients receiving additional estetrol treatment. From these results it can be concluded that the combination of an estrogen suppressant and estetrol can be employed advantageously in postmenopausal breast cancer patients to slow down tumour progression and to treat musculoskeletal pain.

### Example 5

Using the procedure as set forth in example 6, a randomised, double-blinded, placebo-controlled cross-over study is performed to show that additional estetrol treatment of premenopausal breast cancer patients, receiving estrogen suppressant therapy, induces favourable changes in the gene expression profiles from tumours that are obtained by fine needle aspiration and diminishes musculoskeletal pain.
In these premenopausal breast cancer patients ovarian ablation is established by either chemical, surgical, radiotherapeutical means or is reversibly induced by means of a GnRH analog. The results obtained are similar to the clinical outcome reported for postmenopausal breast cancer patients in Exmple 6. From these results it can be concluded that the combination of an estrogen suppressant and estetrol can be employed advantageously in premenopausal breast cancer patients in the treatment of musculoskeletal pain.

## Claims

1. Use of an estrogenic component selected from the group consisting of:
substances represented by the following formula 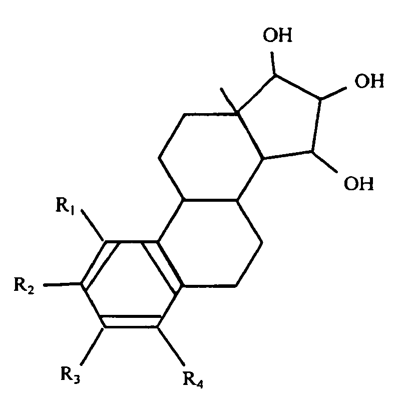
in which formula R₁, R₂, R₃, R₄ independently are a hydrogen atom, a hydroxyl group or an alkoxy group with 1-5 carbon atoms;
precursors capable of liberating a substance according to the aforementioned formula when used in the present method, which precursors are derivatives of the estrogenic substances according to the formula above wherein the hydrogen atom of at least one of the hydroxyl groups has been substituted by an acyl radical of a hydrocarbon carboxylic, sulfonic acid or sulfamic acid of 1-25 carbon atoms; tetrahydrofuranyl; tetrahydropyranal; or a straight or branched chain glycosydic residue containing 1-20 glycosidic units per residue;
and mixtures of one or more of the aforementioned substances and/or precursors;
in the manufacture of a pharmaceutical composition for treating or preventing musculoskeletal pain in a mammal receiving administration of an estrogen suppressant selected from the group consisting of aromatase inhibitors, GnRH analogues, cyclo-oxygenase 2 (COX-2) inhibitors, 17β-hydroxysteroid dehydrogenase type 1 inhibitors, progestogens, anti-estrogens and combinations thereof, said method comprising administering the estrogenic component in an effective amount to prevent said musculoskeletal pain.

2. Use according to claim 1, wherein R₃ represents a hydroxyl group or an alkoxy group.

3. Use according to claim 1 or 2, wherein at least 3 of the groups R₁, R₂, R₃ and R₄ represent hydrogen atoms.

4. Use according to any one of the preceding claims, wherein the method comprises the uninterrupted administration of the estrogenic component during a period of at least 5 days, preferably of at least 30 days.

5. Use according to any one of the preceding claims, wherein the estrogenic component is administered orally, intranasally, transdermally, subcutaneously, intramuscularly, intravenously, intravaginally, intrauterinely, pulmonary, rectally or buccally.

6. Use according to claim 5, wherein the estrogenic component is administered orally, intravaginally, intrauterinely or transdermally.

7. Use according to any one of the preceding claims, wherein the estrogenic component is administered in an amount of at least 1 µg per kg of bodyweight per day, preferably of at least 5 µg per kg of bodyweight per day.

8. Use according to any one of the preceding claims, wherein the estrogen suppressant is administered orally, intranasally, transdermally, subcutaneously, intramuscularly, intravenously, intravaginally, intrauterinely, pulmonary, rectally or buccally.

9. Use according to any one of the preceding claims, wherein the estrogen suppressant is selected from the group consisting of aromatase inhibitors, GnRH analogues, cyclo-oxygenase 2 (COX-2) inhibitors, 17β-hydroxysteroid dehydrogenase type 1 inhibitors, progestogens and combinations thereof.

10. Use according to any one of the preceding claims, wherein the mammal is suffering from breast cancer, uterine cancer, ovarian cancer, endometriosis, uterine fibroids (leiomyomas), benign prostatic hyperplasia and melanoma.

11. Use according to claim 11, wherein the mammal is suffering from breast cancer.

## Patentansprüche

1. Verwendung einer Östrogenkomponente, die aus der Gruppe ausgewählt ist, bestehend aus:
Substanzen der folgenden Formel 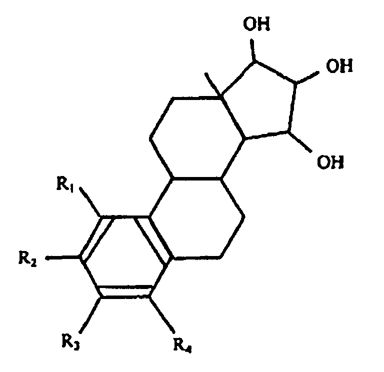
worin R₁, R₂, R₃, R₄ unabhängig voneinander ein Wasserstoffatom, eine Hydroxylgruppe oder eine Alkoxygruppe mit 1 bis 5 Kohlenstoffatomen sind; Vorläuferverbindungen, die eine Substanz der vorstehenden Formel freisetzen können, wenn sie im erfindungsgemäßen Verfahren verwendet werden, wobei die Vorläuferverbindungen Derivate der Östrogensubstanzen der vorstehenden Formel sind, worin das Wasserstoffatom von zumindest einer der Hydroxylgruppen durch einen Acylrest einer Kohlenwasserstoffcarbonsäure, -sulfonsäure oder-sulfaminsäure mit 1 bis 25 Kohlenstoffatomen, Tetrahydrofuranyl, Tetrahydropyranal oder einen geradkettigen oder verzweigten glycosidischen Rest mit 1 bis 20 glycosidischen Einheiten pro Rest ersetzt ist; und
Gemischen von einer oder mehreren vorstehend genannten Substanzen und/oder Vorläuferverbindungen;
bei der Herstellung einer pharmazeutischen Zusammensetzung für die Behandlung oder Verhinderung von Schmerzen der Skelettmuskulatur bei einem Säuger, dem ein Östrogen-Inhibitor verabreicht wird, der aus der Gruppe ausgewählt ist, die aus Aromatase-Inhibitoren, GnRH-Analoga, Inhibitoren für Cyclooxygenase 2 (COX-2), Inhibitoren vom 17β-Hydroxysteroid-Dehydrogenase-Typ 1, Progestogenen, Antiöstrogenen und Kombinationen davon besteht, wobei das Verfahren das Verabreichen der Östrogenkomponente in einer wirksamen Menge umfaßt, um Schmerzen der Skelettmuskulatur zu verhindern.

2. Verwendung nach Anspruch 1, wobei R₃ für eine Hydroxylgruppe oder eine Alkoxygruppe steht.

3. Verwendung nach Anspruch 1 oder 2, wobei zumindest drei der Reste R₁, R₂, R₃ und R₄ für Wasserstoffatome stehen.

4. Verwendung nach einem der vorstehenden Ansprüche, wobei das Verfahren die ununterbrochene Verabreichung der Östrogenkomponente innerhalb eines Zeitraums von mindestens 5 Tagen, vorzugsweise mindestens 30 Tagen umfaßt.

5. Verwendung nach einem der vorstehenden Ansprüche, wobei die Östrogenkomponente oral, intranasal, transdermal, subkutan, intramuskulär, intravenös, intravaginal, intrauterin, pulmunal, rektal oder bukkal verabreicht wird.

6. Verwendung nach Anspruch 5, wobei die Östrogenkomponente oral, intravaginal, intrauterin oder transdermal verabreicht wird.

7. Verwendung nach einem der vorstehenden Ansprüche, wobei die Östrogenkomponente in einer Menge von mindestens 1 µg pro kg Körpergewicht pro Tag, vorzugsweise mindestens 5 µg pro kg Körpergewicht pro Tag verabreicht wird.

8. Verwendung nach einem der vorstehenden Ansprüche, wobei der Östrogen-Suppressor oral, intranasal, transdermal, subkutan, intramuskulär, intravenös, intraviginal, intrauterin, pulmunal, rektal oder bukkal verabreicht wird.

9. Verwendung nach einem der vorstehenden Ansprüche, wobei der Östrogen-Suppressor aus der Gruppe ausgewählt ist, bestehend aus Aromatase-Inhibitoren, GnRH-Analoga, Inhibitoren für Cyclooxygenase 2 (COX-2), Inhibitoren vom 17β-Hydroxysteroid-Dehydrogenase-Typ 1, Progestogenen und Kombinationen davon.

10. Verwendung nach einem der vorstehenden Ansprüche, wobei der Säuger an Brustkrebs, Gebährmutterkrebs, Eierstockkrebs, Endometriose, Uterusfibroiden (Leiomyomen), gutartiger Prostatahyperplasie und Melanomen leidet.

11. Verwendung nach Anspruch 10, wobei der Säuger an Brustkrebs leidet.

## Revendications

1. Utilisation d'un composant oestrogénique choisi dans le groupe constitué par :
des substances représentées par la formule suivante : 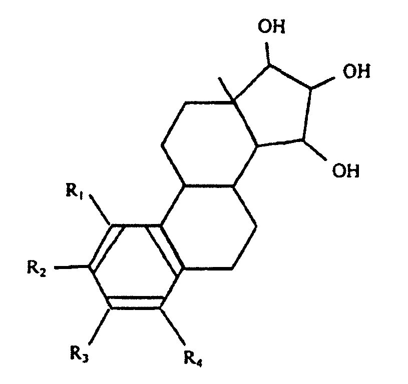
dans laquelle R₁, R₂, R₃, R₄ représentent indépendamment un atome d'hydrogène, un groupement hydroxyle ou un groupement alcoxy ayant 1 à 5 atomes de carbone;
des précurseurs capables de libérer une substance répondant à la formule précitée lorsqu'on les utilise dans le présent procédé, lesquels précurseurs sont des dérivés des substances oestrogéniques répondant à la formule indiquée ci-dessus, dans laquelle l'atome d'hydrogène d'au moins un des groupements hydroxyle a été substitué par un radical acyle d'un hydrocarbure à fonction acide carboxylique, acide sulfonique ou acide sulfamique de 1 à 25 atomes de carbone; un tétrahydrofuranyle; un tétrahydropyranyle; ou un résidu glycosydique à chaîne linéaire ou ramifiée contenant 1 à 20 unités glycosydiques par résidu; et
des mélanges d'une ou plusieurs des substances et/ou d'un ou plusieurs des précurseurs mentionnés ci-dessus;
dans la fabrication d'une composition pharmaceutique pour traiter ou prévenir une douleur musculo-squelettique chez un mammifère auquel est administré un suppresseur d'oestrogènes choisi dans le groupe constitué des inhibiteurs de l'aromatase, des analogues de la GnRH, des inhibiteurs de la cyclo-oxygénase 2 (COX-2), des inhibiteurs de la 17β-hydroxystéroïde déshydrogénase de type 1, des progestogènes, des anti-oestrogènes et de leurs combinaisons, ledit procédé comprenant l'administration du composant oestrogénique en quantité suffisante pour prévenir ladite douleur musculo-squelettique.

2. Utilisation selon la revendication 1, dans laquelle R₃ représente un groupement hydroxyle ou un groupement alcoxy.

3. Utilisation selon la revendication 1 ou 2, dans laquelle au moins 3 des groupements R₁, R₂, R₃ et R₄ représentent des atomes d'hydrogène.

4. Utilisation selon l'une quelconque des revendications précédentes, dans laquelle le procédé comprend l'administration ininterrompue du composant oestrogénique sur une période d'au moins 5 jours, de préférence d'au moins 30 jours.

5. Utilisation selon l'une quelconque des revendications précédentes, dans laquelle le composant oestrogénique est administré par voie orale, intranasale, transdermique, sous-cutanée, intra-musculaire, intraveineuse, intravaginale, intra-utérine, pulmonaire, rectale ou buccale.

6. Utilisation selon la revendication 5, dans laquelle le composant oestrogénique est administré par voie orale, intravaginale, intra-utérine ou transdermique.

7. Utilisation selon l'une quelconque des revendications précédentes, dans laquelle le composant oestrogénique est administré en quantité d'au moins 1 µg par kg de masse corporelle par jour, de préférence, d'au moins 5 µg par kg de masse corporelle par jour.

8. Utilisation selon l'une quelconque des revendications précédentes, dans laquelle le suppresseur d'oestrogènes est administré par voie orale, intranasale, transdermique, sous-cutanée, intramusculaire, intraveineuse, intravaginale, intra-utérine, pulmonaire, rectale ou buccale.

9. Utilisation selon l'une quelconque des revendications précédentes, dans laquelle le suppresseur d'oestrogènes est choisi dans le groupe constitué des inhibiteurs de l'aromatase, des analogues de la GnRH, des inhibiteurs de la cyclo-oxygénase 2 (COX-2), des inhibiteurs de la 17β-hydroxystéroïde déshydrogénase de type 1, des progestogènes et de leurs combinaisons.

10. Utilisation selon l'une quelconque des revendications précédentes, dans laquelle le mammifère souffre d'un cancer du sein, d'un cancer de l'utérus, d'un cancer des ovaires, d'une endométriose, de fibromes utérins (léiomyomes), d'une hyperplasie bénigne prostatique et d'un mélanome.

11. Utilisation selon la revendication 10, dans laquelle le mammifère souffre d'un cancer du sein.
